Europäisches Patentamt

European Patent Office

Office européen des brevets

(19)

(11) Veröffentlichungsnummer : **0 270 729 B1**

(12) # EUROPÄISCHE PATENTSCHRIFT

(45) Veröffentlichungstag der Patentschrift :
23.10.91 Patentblatt 91/43

(51) Int. Cl.$^5$ : **G01N 33/543**

(21) Anmeldenummer : 87105621.4

(22) Anmeldetag : 15.04.87

(54) Laktoferrin enthaltendes Inkubationsmedium für festphasen-immunometrische Verfahren und seine Verwendung.

(30) Priorität : 13.11.86 DE 3638767

(43) Veröffentlichungstag der Anmeldung :
15.06.88 Patentblatt 88/24

(45) Bekanntmachung des Hinweises auf die
Patenterteilung :
23.10.91 Patentblatt 91/43

(84) Benannte Vertragsstaaten :
AT BE CH DE ES FR GB IT LI LU NL SE

(56) Entgegenhaltungen :
DE-A- 2 744 836
GB-A- 2 074 727
CHEMICAL ABSTRACTS, Band 85, Nr. 9, 30
Aug 1976, Columbus, OH (US); R.M.BENNETT
et al., Seite 237, Nr. 59163p
CHEMICAL ABSTRACTS, Band 103, Nr. 19, 11
Nov 1985, Columbus, OH (US); R.W.BEZWODA
et al., Seite 350, Nr. 156619
PATENT ABSTRACTS OF JAPAN, Band 10, Nr.
339 (C-385)[2395], 15 Nov 1986

(73) Patentinhaber : BEHRINGWERKE
Aktiengesellschaft
Postfach 1140
W-3550 Marburg 1 (DE)

(72) Erfinder : Dopatka, Hans-Detlef, Dr.
Heinrich Heine-Str. 9
W-3550 Marburg (DE)
Erfinder : Schmidtberger, Rudolf
Salegrund 1
W-3550 Marburg (DE)

(74) Vertreter : Becker, Heinrich Karl Engelbert, Dr.
et al
HOECHST AKTIENGESELLSCHAFT Central
Patent Department P.O. Box 80 03 20
W-6230 Frankfurt am Main 80 (DE)

## Beschreibung

Die Erfindung betrifft ein Laktoferrin enthaltendes Inkubationsmedium für festphasen-immunometrische Tests und die Verwendung dieses Mediums.

Für festphasen-immunometrische Tests, beispielsweise ELISA, müssen die erforderlichen Inkubationsmedien ("Pufferlösungen", "Inkubationsmilieus") so zusammengesetzt sein, daß die unspezifische Bindung von Begleitsubstanzen der Probe an die Festphase verhindert wird. Dafür bekannte Zusätze sind Proteine wie Albumin, Kasein, Gemische von Proteinen wie tierische Seren, hydrolysierte Gelatine oder deren Derivate, sowie Tenside.

Mit solchen Zusätzen versehene Inkubationsmedien können falsche Meßwerte nicht verhindern, die besonders beobachtet werden, wenn Proben erhitzt worden sind. Das Erhitzen ist zweckmäßig, um die Infektiosität der Proben herabzusetzen, die beispielsweise durch HIV (humanes Immundeficiency Virus) gegeben sein kann.

Es bestand daher die Aufgabe, ein Inkubationsmedium zu finden, bei dessen Verwendung durch Erhitzen der Probe verursachte falsche Werte nicht auftreten.

Es wurde überraschenderweise gefunden, daß ein Inkubationsmedium, das Laktoferrin enthält, diese Aufgabe löst.

Gegenstand der Erfindung ist ein Inkubationsmedium für festphasen-immunometrische Tests enthaltend Lactoferrin als Mittel zur Verhinderung der unspezifischen Bindung von Begleitsubstanzen der Probe.

Gegenstand der Erfindung ist weiterhin die Verwendung von Lactoferrin als Mittel zur Verhinderung der unspezifischen Bindung von Begleitsubstanzen einer Probe in einem festphasen-immunometrischen Test.

Vorteilhafte Ausgestaltungen der Erfindung sind in den Ansprüchen näher gekennzeichnet.

Ein Inkubationsmedium im Sinne der Erfindung ist die flüssige Phase eines festphasen-immunometrischen Test, in der die immunchemische Reaktion abläuft.

Laktoferrin wird in einer Konzentration von 0.05 bis 20 g/l zugegeben. Bevorzugt wird eine Konzentration von 0.15-0.25, besonders 0.2 g/l.

Das Laktoferrin kann an sich bekannten Inkubationsmedien oder Pufferlösungen für festphasen-immunometrische Tests zugesetzt werden. Eine davon ist beispielsweise die in der DE-A-27 448 36 auf Seite 24 beschriebene Lösung, die außer Puffersubstanz 5 g/l Rinderserumalbumin, 5 g/l Polyoxiethylen-(20)-sorbitanmonolaurat ((R)Tween 20) in phosphat-gepufferter physiologischer Kochsalzlösung (PBS) enthält. Als weiteres Beispiel sei die in DE-A-31 15 115 auf Seite 10 beschriebene Lösung angeführt, ein 0.2 mol/1 $NaH_2PO_4$-Puffer, pH 6.5, der 2 g/l Rinderserumalbumin und 200 ml/l normales Ziegenserum enthält. Eine bevorzugte Pufferlösung ist im Beispiel 1 verwendet.

Ein solches Medium kann vorteilhaft ein weiteres, an sich bekanntes Protein, vorzugsweise Kasein, und/oder ein Hydrolyseprodukt von Gelatine oder hydrolysierte und chemisch behandelte Gelatine inthalten.

Das erfindungsgemäß zu verwendende Laktoferrin ist erhältlich aus Milch eines Säugers, indem die Milch entrahmt, die entrahmte Milch auf einen pH-Wert von 3.5-4.5 eingestellt, die entstandene Proteinfällung abgetrennt und aus dem Überstand das Laktoferrin durch Chromatographie an einem Ionenaustauscher abgetrennt und isoliert wird.

Laktoferrin wird aus Milch, vorzugsweise aus Kuhmilch isoliert. In einem bevorzugten Verfahren zur Gewinnung eines für den erfindungsgemäßen Zweck geeigneten Laktoferrins wird die Milch entfettet, indem sie beispielsweise zentrifugiert wird. Von den sich bildenden zwei Phasen wird die obere abgetrennt und verworfen. Die untere Phase wird mit einer Säure auf pH 3.5-4.5 eingestellt. Dabei entsteht ein Präzipitat, welches beispielsweise durch Zentrifugation sedimentiert wird und verworfen wird. Der Überstand wird durch Verdünnen mit Wasser auf eine Leitfähigkeit von 6-7 mS/cm eingestellt. Dann wird gequollener und mit einem Puffer von pH 4-5.5 und einer Leitfähigkeit von vorzugsweise 6-7 mS/cm equilibrierter Kationenaustauscher, beispielsweise CM-Zellulose oder SP-(R)Sephadex, eingetragen. Die Suspension wird filtriert und der Rückstand mehrmals mit demselben Puffer gewaschen, dann darin suspendiert und in eine Säule gepackt. Es wird mit einem Gradienten von vorzugsweise 0-1 mol/l NaCl bei pH 5 eluiert. Die Fraktion, die im Bereich von 500-650 mmol/l NaCl als einheitlicher bei 280 nm gemessener Peak eluiert wird, enthält das für das Inkubationsmedium besonders geeignete Laktoferrin. Dieses Laktoferrin erscheint in einer SDS-PAGE Elektrophorese als Bande mit einem Molekulargewicht von 80000±3000.

Bei Chromatographie an einem Anionenaustauscher, beispielsweise an DEAE- oder QAE-(R)Sephadex, oder an DEAE-Zellulose wird der Überstand aus der oben beschriebenen Fällung bei pH 3.5-4.5 auf pH 6.5-7.5 und eine Leitfähigkeit von 6-8 mS/cm eingestellt und auf eine Säule gefüllt mit dem Ionenaustauscher gegeben, der zuvor mit einem Puffer von pH 6.5-7.5 und einer Leitfähigkeit von 6-8 mS/cm equilibriert worden war. Das Laktoferrin erscheint dann im Durchlauf oder es wird als erster Peak bei Elution mit einem NaCl-Gradienten eluiert.

Das Laktoferrin muß bei der Inkubation der Probe, bzw. des Analyten mit den an die Festphase gebundenen Reaktanten vorhanden sein. Es kann der Lösung, die den Analyten enthält oder auch der Lösung, bzw. dem Puffer, mit dem der Analyt verdünnt wird, zugesetzt werden. Es kann aber auch in gelöster Form mit dem an die Festphase gebundenen Reaktanten in Kontakt gebracht und/oder auf die Festphase getrocknet werden, bevor diese mit dem Analyten in Kontakt gebracht wird.

Als an Festphasen gebundene Reaktanten kommen in Frage die Komponenten eines bioaffinen Bindungssystems, die einen solchen Analyten zu binden vermögen. Im Falle eines immunologischen Bindungssystems sind diese Reaktanten entweder Antigene oder Antikörper. Festphasen-immunometrische Tests können sowohl kompetitive, als auch zweiseiten-immunometrische (Sandwich) oder auch indirekte Tests sein. Im letztgenannten Fall ist der Analyt ein Antikörper, der Festphasenreaktant das entsprechende Antigen, wobei die Menge des an die Festphase gebundenen Antikörpers durch einen zweiten, markierten Antikörper bestimmt wird.

Das Testsystem kann ein Ein- oder Mehrschrittassay sein.

Das Laktoferrin verhindert die Bindung von solchen Bestandteilen der Probe, die nicht zu dem jeweiligen bioaffinen Bindungssystem gehören. Es vermeidet somit falsche Ergebnisse beim Nachweis und bei der Bestimmung des Analyten.

Die folgenden Beispiele erläutern die Erfindung und belegen die Zweckmäßigkeit des Laktoferrin als Bestandteil des Inkubationsmediums.

Die Beispiele sollen zeigen, daß Laktoferrin in verschiedenen Inkubationsmedien in unterschiedlichen Mengen eingesetzt brauchbar ist.

Beispiel 1

1. Isolierung von Laktoferrin

20 l Kuhmilch wurden 30 min bei 3400xg zentrifugiert, das Milchfett im Überstand entfernt. Geringe Anteile unlöslicher Bestandteile wurden als Sediment abgetrennt und ebenfalls verworfen.

Der pH-Wert der entfetteten Milch wurde durch Zugabe von Salzsäure auf pH 4.0 eingestellt. Das dabei auftretende Proteinpräzipitat wurde durch Zentrifugieren bei 3400 x g entfernt.

Der Überstand, der nach Einstellen von pH 5.0 mit Natronlauge eine Leitfähigkeit von 12 mS/cm bei 20°C hatte, wurde mit Wasser auf 7 mS/cm verdünnt und dann mit 200 ml des gequollenen Ionenaustauschers Carboxymethylcellulose CM 32 der Fa. Whatman versetzt und 4 h bei Raumtemperatur gerührt. Der Ionenaustauscher war vorher mit 50 mmol/l Natriumacetatpuffer, pH 5.0 equilibriert worden. Anschließend wurde der Ionenaustauscher über eine Filternutsche abfiltriert und in dem Natriumacetatpuffer suspendiert und in eine Chromatographiesäule überführt. Die Säule wurde anschließend mit 400 ml des vorher beschriebenen Acetatpuffers gespült. Die an den Kationenaustauscher gebundenen Proteine wurden mit einer Lösung von 1 mol/l Natriumchlorid eluiert.

Das Eluat wurde mit 1 normaler Natronlauge auf pH 5.0 eingestellt und durch Dialyse in 50 mmol/l Natriumacetat, pH 5.0 überführt. Das Proteingemisch wurde anschließend an 200 ml Carboxymethylcellulose CM 32 rechromatographiert. Die Elution der Proteine erfolgte durch einen linearen Gradienten, der von 50 mmol/l Natriumacetat pH 5 bis 50 mmol/l Natriumacetat, pH 5 und 1 mol/l Natriumchlorid reichte.

Der 4. Peak des Elutionsdiagrammes, dessen Lösung eine Leitfähigkeit von 18-20 mS/cm hatte, wurde als eine Fraktion gesammelt, durch Ultrafiltrieren konzentriert und an [R]Sepharose AcA 34 gelfiltriert, die mit PBS enthaltend 0.5 g/l Natriumazid equilibriert war. Die 1.2 g Protein ausmachende Hauptkomponente der Gelfiltration enthielt das Laktoferrin.

2. Vergleich von Probenverdünnungspuffer enthaltend Laktoferrin mit Probenverdünnungspuffer ohne Zusatz

Für das festphasen-immunometrische Verfahren wurde das ELISA-Besteck Enzygnost[R]Anti HSV der Behringwerke verwandt. Bei der Testdurchführung wurde der in der Packungsbeilage beschriebenen Arbeitsweise gefolgt, die wie folgt kurz dargestellt ist.

Die Proben wurden mit Probenverdünnungspuffer (137 mmol/l NaCl, 7.247 mmol/l $Na_2HPO_4.2H_2O$ und 2.720 mmol/l $KH_2PO_4$ mit den Zusätzen von 2.7 mmol/l KCl, 0.4 mmol/l $MgCl_2$, 3 mmol/l $NaN_3$, 10 ml/l foetalem Kälberserum und 40 ml/l [R]Tween 20) ohne oder mit Zusatz von 0.2 g/l Laktoferrin im Verhältnis 1:25 anstatt wie empfohlen 1:44 verdünnt und mit in den Näpfchen einer Microtestplatte fixierten Antigenen zur Inkubation gebracht, die nicht gebundenen Antikörper ausgewaschen, über eine Konjugatlösung ein Enzym an den gebildeten Antikörper/Antigen-Komplexen gebunden, die überschüssige Konjugatlösung ausgewaschen, mittels einer chromogenen Substratlösung die Einfärbung über das Enzym bewirkt und anschließend mit einer Stopp-Lösung die Reaktion beendet. Die so hergestellten, in Abhängigkeit vom Gehalt der virusspezifischen Antikörper eingefärbten Proben wurden dann bewertet, indem ihre Extinktion (E) photometrisch bei 405 nm bestimmt wurde.

Jeweils ein Teil von 9 Probandenseren wurde 30 min auf 56°C erhitzt. Der andere Teil blieb unbehandelt.

Es wurde gefunden, daß bei erhitzten Proben in Verdünnungspuffer ohne Laktoferrin vor allem sehr hohe Extinktionen in den Kontroll-Näpfchen gemessen wurden, aber auch erhöhte Extinktionen in den antigen-beschichteten. Dies führte zu 5 falsch-negativen Ergebnissen. Die Kontroll-Näpfchen sind mit der Präparation nicht virusinfizierter Zellen, die für die Gewinnung der Viren verwendet werden, behandelt ("beschichtet"). Wenn die 9 erhitzten Proben unter Verwendung des Laktoferrin enthaltenden Verdünnungspuffers getestet wurden, wurde kein falschnegativer Wert gefunden. Die nicht erhitzten Proben wurden unter Verwendung des laktoferrinfreien Verdünnungspuffers geprüft. Die Ergebnisse dienten als Kontrolle der Ergebnisse mit den erhitzten Seren.

Beispiel 2

Die Wirkung eines Zusatzes von Laktoferrin wurde in einem Einschritt-Sandwich-Test geprüft. Wenn in einem solchen Test für HBsAg (Hepatitis B surface antigen) 7,5 g/l Laktoferrin zugegeben wurde, wurden alle von 14 nichterhitzten kritischen Proben richtig negativ gefunden, während ohne Zusatz von Laktoferrin alle als falsch positiv bestimmt worden waren.

Beispiel 3

Die Wirkung eines Zusatzes von Laktoferrin wurde in einem Dreischritt-Sandwich-Test zum Nachweis von Influenza A-Antigen geprüft. Laktoferrin wurde im zweiten und dritten Schritt dieses festphasen-immunochemischen Verfahrens eingesetzt.

Wenn in diesen beiden Schritten zuerst ein gebrauchsverdünntes Antikörper/Biotin-Konjugat und danach ein gebrauchsverdünntes Streptavidin/POD-Konjugat, enthaltend Laktoferrin (mehr als 0,05 g/l), inkubiert wurden, ließen sich die unspezifischen Reaktionen bezüglich Background und Influenza B-Fremdantigen um über 80 % senken. Stellvertretend ist hier ein ELISA-Ergebnis angeführt, gemessen bei 450 nm:

Unspezifische Bindung ohne Laktoferrin = 2,892 E.

Unspezifische Bindung mit Laktoferrin = 0,231 E.

**Patentansprüche**

**Patentansprüche für folgende Vertragsstaaten: AT BE CH DE FR GB IT LI LU NL SE**

1. Inkubationsmedium für festphasen-immunometrische Tests enthaltend Laktoferrin als Mittel zur Verhinderung der unspezifischen Bindung von Begleitsubstanzen der Probe.

2. Inkubationsmedium nach Anspruch 1, dadurch gekennzeichnet, daß das Laktoferrin in dem Konzentrationsbereich von 0.05 - 20 g/l vorliegt.

3. Inkubationsmedium nach Anspruch 1, dadurch gekennzeichnet, daß es Laktoferrin enthält, das erhältlich ist aus Milch eines Säugers, indem

a) die Milch entrahmt,

b) die entrahmte Milch auf einen pH-Wert von 3.5 - 4.5 eingestellt,

c) die bei (b) entstehende Proteinfällung abgetrennt und

d) aus dem bei (c) entstehenden Überstand das Laktoferrin durch Chromatographie an einem Ionenaustauscher abgetrennt und isoliert wird.

4. Inkubationsmedium nach Anspruch 1 enthaltend ein Puffersalz, Laktoferrin und gegebenenfalls ein weiteres Protein und gegebenenfalls ein Tensid und gegebenenfalls ein Neutralsalz.

5. Inkubationsmedium nach Anspruch 1 enthaltend ein Puffersalz, Laktoferrin, Rinderserumalbumin, ein Tensid und gegebenenfalls ein Neutralsalz.

6. Inkubationsmedium nach Anspruch 1 enthaltend ein Puffersalz, Laktoferrin, ein Hydrolyseprodukt von Gelatine oder hydrolysierte und chemisch behandelte oder chemisch behandelte und hydrolysierte Gelatine und ein Tensid und gegebenenfalls ein Neutralsalz.

7. Verwendung von Laktoferrin als Mittel zur Verhinderung der unspezifischen Bindung von Begleitsubstanzen einer Probe in einem festphasen-immunometrischen Test.

8. Verwendung eines Inkubationsmediums nach Anspruch 1 in festphasen-immunometrischen Tests zum Nachweis und zur Bestimmung eines Antikörpers in einer biologischen Flüssigkeit, die erhitzt worden war.

9. Verwendung eines Inkubationsmediums nach Anspruch 1 in festphasen-immunometrischen Tests zum Nachweis und zur Bestimmung eines Antigens in einer biologischen Flüssigkeit, die erhitzt worden war.

10. Verwendung eines Inkubationsmediums nach Anspruch 1 in festphasen-immunometrischen Tests zum Nachweis und zur Bestimmung eines Antikörpers oder eines Antigens in einer biologischen Flüssigkeit, die aggregierte Bestandteile in gelöster Form enthält.

**Patentanspruch für folgenden Vertragsstaat: ES**

1. Verfahren zur Herstellung eines Inkubationsmedium für festphasen-immunometrische Tests, enthaltend Laktoferrin als Mittel zur Verhinderung der unsperifischen Bindung von Begleitsubstanzen der Probe, dadurch gekennzeichnet, daß Lactoferrin sowie ein Puffersalz und gegebenenfalls ein weiteres Protein, das gegebenenfalls abgebaut und chemisch vernetzt ist, und gegebenenfalls ein Tensid in einer Menge vermischt werden, daß beim Auflösen in Wasser das Laktoferrin in einem Konzentrationsbereich von 0.05 bis 20 g/l vorliegt.

## Claims

### Claims for the following Contracting States:
### AT BE CH DE FR GB IT LI LU NL SE

1. An incubation medium for solid-phase immunometric assays containing lactoferrin as agent for preventing non-specific binding of concomitant substances in the sample.

2. An incubation medium as claimed in claim 1, wherein the lactoferrin is present in the concentration range 0.05-20 g/l.

3. An incubation medium as claimed in claim 1, which contains lactoferrin which can be obtained from milk of a mammal by
   a) removing the cream from the milk,
   b) adjusting the milk, from which the cream has been removed, to a pH of 3.5-4.5,
   c) removing the protein precipitate which has been produced in (b), and
   d) removing and isolating the lactoferrin from the supernatant produced in (c) by chromatography on an ion exchanger.

4. An incubation medium as claimed in claim 1 containing a buffer salt, lactoferrin and, where appropriate, another protein and, where appropriate, a surfactant and, where appropriate, a neutral salt.

5. An incubation medium as claimed in claim 1 containing a buffer salt, lactoferrin, bovine serum albumin, a surfactant and, where appropriate, a neutral salt.

6. An incubation medium as claimed in claim 1 containing a buffer salt, lactoferrin, a gelatin hydrolysis product or hydrolyzed and chemically treated, or chemically treated and hydrolyzed, gelatin, and a surfactant and, where appropriate, a neutral salt.

7. The use of lactoferrin as agent for preventing non-specific binding of concomitant substances in a sample in a solid-phase immunometric assay.

8. The use of an incubation medium as claimed in claim 1 in solid-phase immunometric assays for the detection and determination of an antibody in a biological fluid which has been heated.

9. The use of an incubation medium as claimed in claim 1 in solid-phase immunometric assays for the detection and determination of an antigen in a biological fluid which has been heated.

10. The use of an incubation medium as claimed in claim 1 in solid-phase immunometric assays for the detection and determination of an antibody or an antigen in a biological fluid which contains aggregated constituents in dissolved form.

### Claims for the following Contracting States:
### ES

1. A process for the preparation of an incubation medium for solid-phase immunometric assays, containing lactoferrin as agent for preventing non-specific binding of concomitant substances in the sample which comprises mixing lactoferrin and a buffer salt and, where appropriate, another protein which has, where appropriate, been degraded and chemically crosslinked, and, where appropriate, a surfactant in an amount such that, on dissolution in water, the lactoferrin is present in a concentration range of from 0.05 to 20 g/l.

## Revendications

### Revendications pour les Etats contractants
### suivants: AT BE CH DE FR GB IT LI LU NL SE

1. Milieu d'incubation pour méthodes immunométriques en phase solide, renfermant de la lactoferrine comme agent empêchant la fixation non spécifique de substances accompagnant l'échantillon.

2. Milieu d'incubation selon la revendication 1, caractérisé en ce que la lactoferrine est présente à une concentration de 0,05 à 20 g/l.

3. Milieu d'incubation selon la revendication 1, caractérisé en ce qu'il contient de la lactoferrine, que l'on obtient à partir du lait d'un mammifère, en
   a) écrémant ce lait,
   b) ajustant le pH du lait écrémé à 3,5-4,5,
   c) séparant les protéines précipitées en (b), et
   d) séparant et isolant la lactoferrine d'avec le résidu formé en (c), par chromatographie sur échangeur d'ion.

4. Milieu d'incubation selon la revendication 1, renfermant un sel tampon, de la lactoferrine, éventuellement une autre protéine, éventuellement un agent tensioactif et éventuellement un sel neutre.

5. Milieu d'incubation selon la revendication 1, renfermant un sel tampon, de la lactoferrine, de la sérum-albumine bovine, un agent tensioactic et éventuellement un sel neutre.

6. Milieu d'incubation selon la revendication 1, renfermant un sel tampon, de la lactoferrine, un produit d'hydrolyse de la gélatine ou de la gélatine hydrolysée et chimiquement traitée ou bien de la gélatine chimiquement traitée et hydrolysée, ainsi qu'un agent tensioactif et éventuellement un sel neutre.

7. Emploi de la lactoferrine comme agent pour empêcher la fixation non spécifique de substances accompagnant un échantillon, dans une méthode immunométrique en phase solide.

8. Emploi d'un milieu d'incubation selon la revendication 1, dans des méthodes immunométriques en phase solide, pour identifier et doser un anticorps dans un liquide biologique qui a été chauffé.

9. Emploi d'un milieu d'incubation selon la revendication 1, dans des méthodes immunométriques en phase solide, pour identifier et doser un antigène dans un liquide biologique qui a été chauffé.

10. Emploi d'un milieu d'incubation selon la revendication 1, dans les méthodes immunométriques en phase solide, pour identifier et doser un anticorps ou un antigène dans un liquide biologique qui renferme des constituants agrégés à l'état dissous.

**Revendication pour l'Etat contractant suivant: ES**

1. Procédé de préparation d'un milieu d'incubation pour méthodes immunométriques en phase solide, renfermant de la lactoferrine comme agent empêchant la fixation non spécifique de substances accompagnant l'échantillon, caractérisé en ce que l'on mélange de la lactoferrine, un sel tampon, éventuellement une autre protéine, qui a été éventuellement dégradée et réticulée chimiquement, et éventuellement un agent tensiocatif, en des proportions telles que la loctoferrine, après dissolution dans l'eau, est présente à une concentration de 0,05 a 20 g/l.